# EUROPEAN PATENT APPLICATION

(11) **EP 4 356 958 A1**
(43) Date of publication of application: **24.04.2024**
(21) Application number: 22202000.0
(22) Date of filing: 17.10.2022
(51) Int. Cl.: A61N 2/00, A61N 2/02

(54) **MAGNETIC FIELD MEDICAL DEVICE CONSTITUTED BY INTELLIGENT ANTENNAS**

(71) Applicant: Led S.p.A., 03100 Frosinone (FR) (IT)
(72) Inventor: MAUTI, Marco, I-03100 Frosinone (FR) (IT)
(74) Representative: Fiammenghi, Eva

(57) **Abstract**

Magnetic field medical device constituted by intelligent antennas comprising:
- a grid-powered base (1) with keyboard made of polycarbonate, function keys and backlit screen (4);
- a pair of intelligent antennas (3) power supplied with rechargeable internal battery;
- an accessory (2) to which the provided textile accessories are connected;
- a lithium battery up to 3.7 V 900 mAh, duration of autonomy up to 90 minutes and in continuous supply;
- at least two connection cables with said textile accessories (respectively 1.5 and 2.5 m).

## Description

### Field of the art

The present invention operates in the context of medical electronic apparatuses for assisted physiotherapy. In particular, the product is situated within the instrumental physical therapy, a branch of physiotherapy, which uses the electromagnetic energy for stimulating the regeneration of the tissues. Indeed, the benefits of use of magnetotherapy are above all at the articular level (arthritis, arthrosis, osteoporosis, bone fractures, etc.), at the muscular/tendon level (lumbago, tendinitis, distortions, rheumatic pain, etc.) and at vascular level (phlebitis, varicose veins, edemas, sores, scars, etc.).

### Prior art

The first observations of the interactions of the magnetic fields on human health are of very ancient origins, and even traceable to Hippocrates and the ancient Egyptians. The first systematic studies and the observations of the relationship between magnetic fields and living organisms instead started towards the end of the 1500s. But only with the scientific discoveries and the technological progresses of the last century was there the extraordinary impulse towards the knowledge and the possible applications of electromagnetic therapy in medicine, above all due to Russian, American, Japanese and French studies (Danielewsky, Basset, Pilla, Fukuda, Yasuda, D'Arsonval, Fellus; etc.). For these reasons, the validity of the application of the magnetic fields in therapeutic field was officially recognized in the medical field only in recent decades. Numerous devices were developed in recent years to be employed in the field of magnetotherapy, such as the patented product US5813971 and many others. What has not been extensively studied, however, is the environmental impact tied to the energy necessary for their production, as well as to all the apparatuses necessary for their use. An intelligent wireless technology would allow a more efficient use of the energy, also in the case of recharging conventional batteries, ensuring that the device with completely charged battery would not be left charging, thus preventing the automatic waste of the stand-by devices and a reduction of the costs ascribable to the energy by an entire community. In addition, an update on the technology currently in use is necessary. Indeed, products are present that employ the use of antennas such as Magnetofield, but these are multiple antennas that must carry out the same program, without being able to execute independent programs, thus limiting the effectiveness of the therapy.

Hence there is no doubt that such solutions only partly confront the critical aspects set forth above.

### Description of the invention

According to the present invention, magnetotherapy machinery is attained for treating osteo-articular pathologies, effectively solving the abovementioned problems. Magnetotherapy is a form of physiotherapy which uses the electromagnetic waves for therapeutic purposes. The device presented works both at low frequency (for frequencies from 1 to 100 Hz) and at high frequency (for frequency within the radio wave range between 1 and 300 MHz). The low frequency pulses (BF) are capable of stimulating a greater stimulation of calcium, a very important factor since the bones, being strengthened, are much less subject to risks of fracture, onset of degenerative diseases and osteoporosis. The high frequency pulses (AF), instead, improve the blood circulation and are capable of stimulating the production of endorphins by the neurovegetative system, with consequent reduction of the pain associated with the various pathogenic states and an effective anti-inflammatory action. In light of the described aspects, we can conclude that it is possible to treat both acute and chronic pathologies, contributing to an effective treatment and/or improvement action for the disturbances/pain associated therewith. The benefits of magnetotherapy are therefore multiple; several are listed hereinbelow:
- anti-inflammatory action (AF);
- analgesic/antipain effect (AF) by means of stimulation of the production of endorphins;
- effect on the bone tissue (BF/AF) by means of an increase of the bone strength, an improvement of the osteogenesis, an increase of the mineralization and an acceleration in the calcification of the fractures;
- vaso-active action (AF) by means of an increase of the peripheral blood flow and of the speed of blood flow, an increase vascular spraying, an improvement of the oxidation and reduction of the liquid accumulation;
- antiedematous and tissue repair action (AF) by means of the acceleration of the scarring of wounds, sores and of the healing process of the soft tissues.

Therefore, the benefits of the use of magnetotherapy are above all at the articular level (arthritis, arthrosis, osteoporosis, bone fractures, etc.), at the muscular/tendon level (lumbago, tendinitis, distortions, rheumatic pain, etc.) and at the vascular level (phlebitis, varicose veins, edemas, sores, scars, etc.). The present invention has the possibility of working at low frequency (from 6 at 100 Hz) and at high frequency (from 100 at 5000 Hz). All the arranged programs are managed by microprocessor and have automatic scanning: it is possible to select the therapy connected to the pathology present and the apparatus directly emits the electromagnetic field. In addition, it does not have to be connected to the power grid, except when it is necessary to recharge the internal battery. The application sessions have a recommended duration of 15/20 minutes, that can be prolonged according to the pathology and the user needs, usually once a day and for an overall duration variable between 15 and 30 days. The treatment periods can be repeated cyclically with an interval of about 20/30 days. The present invention integrates an innovative system of double therapy simultaneous with variable multifrequency and bipolar magnetic pulses. This union assists the treatments in being even more effective, regenerating the organism and improving the microcirculation which activates the smallest blood vessels. Therefore, it reduces the inflammation in the affected area and improves the performances of the body cells, significantly eliminating the pain and increasing the power of body healing. The magnetotherapy device is constituted by three distinct parts (also termed local devices):
- a base;
- a pair of antennas;
- an accessory.

In addition, it is underlined that this is a wireless apparatus with communication range between base and local devices equal to at least 10 meters, in a normal living/housing condition. Hence, the associated local device do not have to be connected to the base apparatus so to be able to operate. The program that one intends to use is loaded thereon; the antennas are then applied directly to the body, while the textile accessories are connected to the accessory. This allows being able to execute treatments in places and in situations that are very different from each other, and in any case also in a manner completely free from the base. The power supplier has the single role of increasing the base and recharging the battery of the local devices (when it is inserted, therapies therefore cannot be carried out). The device, moreover, is equipped with a lithium battery up to 3.7 V 900 mAh, duration of autonomy up to 90 minutes and in continuous supply. The characteristics of the product are listed in Table 1, which is reported hereinbelow.

**Table 1. Characteristics of the product.**

| | |
|---|---|
| Power supply | 100-240 Vac |
| Power supply frequency | 6-100 Hz (BF) 100-5000 Hz (AF) |
| Power | up to 200 Gauss |

Table 1. Characteristics of the product.

The combination of the above-listed characteristics plays a fundamental role and each of these contributes to the attainment of an optimal highly innovative result. Hence, in addition the functions of the technology, the present invention allows combining the therapeutic effectiveness of the technology with an ease of use given by the presence of an interface that is highly intuitive and clear, which allows creating personalized paths and saving them in a memory so to be able to conduct statistics and analysis on the progresses of the therapy, associated with each clinical case. Indeed, at least 100 predefined protocols are available, stored in the database. In addition, it is possible to enlarge the spectrum of personalized protocols based on the needs of each clinical case, being able to add at least 200 protocols that can be archived in the internal memory and at least 200 in a suitable Smart card. In addition it should be underlined that with respect to the technologies that are on the market, the dimensions of the apparatus are reduced and this allows an easy transportability. In addition, the machinery is equipped with a user-friendly operating system, which at its interior contains a guided protocol for body districts, a protocol per pathology, an anti-edema protocol and a standard protocol. Also present is a characteristic that allows carrying out an automatic calculation of the energy emitted as a function of the selected parameters. Alarms and safety equipment are also associated with the apparatus, as reported hereinbelow:
- acoustic signal for therapy start and for every second of treatment (deactivatable);
- calendar and clock;
- timer to be set at the start of each therapy in order to define the duration, expressed in minutes;
- language change (up to 6 available languages);
- warnings and alarms relative to the state of the machine;
- emergency key for stopping the machinery;
- patient database;
- duty cycle adjustable from 10% to 100%;
- interlocking;
- function for controlling the emitted power;
- self-diagnosis, which provides an automatic diagnostic control of the present complete functions of the electronic unit and of the connected accessories and encoded indication of the malfunctions encountered.

The warnings and alarms relative to the state of the machinery make reference in particular to the temperature of the environment where the machinery is preserved, which must fall within the following pre-established regulations: optimal temperature comprised between +15°C and +35°C, humidity between 45% and 75% and atmospheric pressure between 860 hPa and 1060 hPa. Additionally, a sensor is present which detects the presence of a heat source in proximity to the machinery (such as solar rays or heaters) and a consequent alarm for instantaneous machine turn-off due to overheating danger. In addition, the machinery is provided with an antifreeze device in order to prevent the temperature of the premises from following below +5°C. In addition, the function of being able to execute the updating of the system by means of USB ports. The device is also associated with an application for smartphones and tablets which connects the professional to an international network of doctor colleagues. Said application allows the professional to take photos before and after, modifying them into a format useful for easy sharing. Hence, it offers a platform for keeping up-to-date with patients, colleagues and scientific committees throughout the world and provides updates on protocols and events organized by the company. In addition, it has been made available both on iOS and on Android. The advantages offered by the present invention are evident in light of the description set forth up to now and will be even clearer due to the enclosed figures and to the relative detailed description.

### Description of the figures

The invention will be described hereinbelow in at least one preferred non-limiting embodiment with the aid of the enclosed figures, in which:

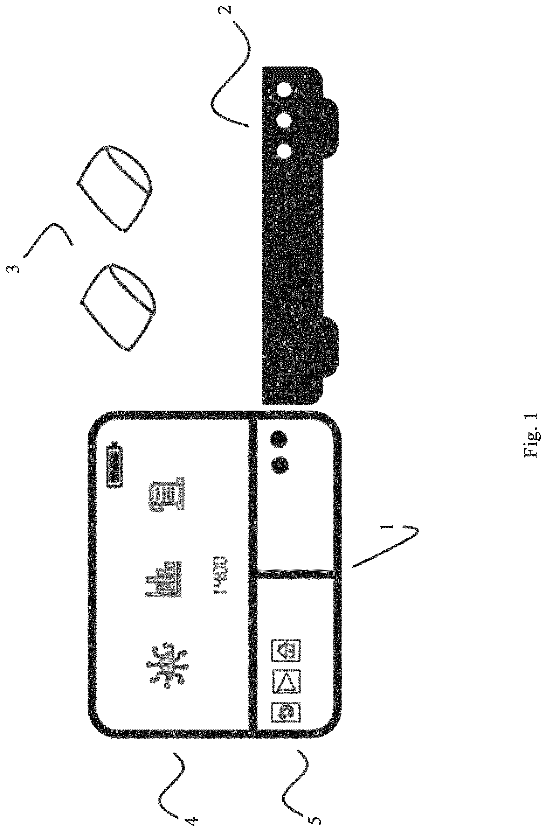

- FIGURE 1 shows all the constituent elements of the device. The present invention is composed of three local devices:
   1. a base 1, which manages the programs, but does not execute treatments. It is power supplied with keyboard made of polycarbonate on which the buttons 5 are present, which allow carrying out immediate actions such as returning to the main home, modifying the base settings, command for activating or stopping device;
   2. a pair of intelligent antennas 3 with elastic bands for positioning them, power supplied with rechargeable internal battery;
   3. an accessory 2 to which the provided textile accessories are connected.
   The screen 4, which is a backlit touchscreen 7-9", allows selecting different functions:
   A) protocols predefined for body districts (standard, rapid and aimed depending on the pathology);
   B) programs that can be edited and stored in order to personalize the parameters depending on the treatment;
   A) database of the parameters personalized for each patient. Such data can be easily recovered depending on the needs;
   B) analysis and statistics of the results obtained upon varying the parameters;
   C) personal data of the patients with relative physical characteristics (weight, height, particular signs);
   D) setting for activating the machinery;
   E) option for selecting the preferred language (at least 6 languages);
   F) calendar and clock;
   G)warnings and alarms of the state of the machine.

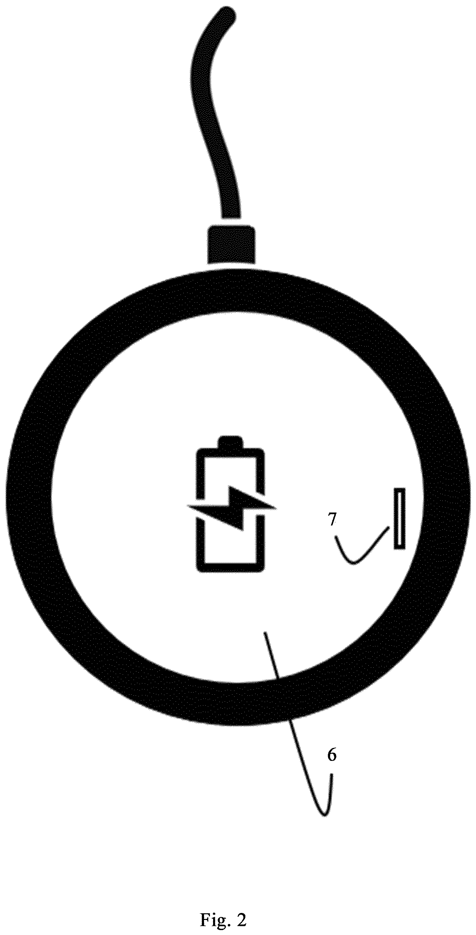

- FIGURE 2 illustrates the recharge station of the device represented in FIG. 1. The device must be positioned above the central recharge surface 6. This is a wireless recharge station of variable dimensions comprised between 58÷64 cm with rather limited diameter and weight comprised between 2÷4 kg. Said recharge station allows the device to be recharged when it is not used, in only 4/5 hours. This allows the total absence of cables and an easy handling during the treatments in places and situations that are quite different from each other. In addition, the recharge station has a LED 7 that indicates when the device is being charged.

### Detailed description of the invention

The present invention will now be illustrated as a merely non-limiting or non-binding example, with reference to the figures which illustrate several embodiments relative to the present inventive concept. With reference to FIG. 1, the machinery of the present invention is shown, through which it is possible to monitor the use of the magnetotherapy. The screen 4 is present thereon, which shows the progression of the therapy underway, the updated data, calendar and clock and possible alarms and/or warnings. Through the screen 4 it is also possible to select the protocol and the program most suitable for the clinical case under examination, the planning of the therapy and all the abovementioned features. With the aid of the buttons 5, it is possible to set the parameters and activate and/or interrupt the therapy. The base 1 automatically programs the antennas 3, one as positive, backlit with red color, and the other as negative, backlit in green color. At the start of the treatment program, they are synchronized with each other, as work times and parameters. The accessory 2 operates autonomously, independent of the antennas 3. By means of the wireless communication between the three local devices, the selected program and the battery recharge state are transmitted. Such information is also displayable not only on the screen 4, but also by means of the application. In the wireless communication, moreover, the synchronization is maintained. By way of example, the operation of the device occurs by means of the passages listed below:
- Connecting the antennas 3 and the accessory 2 in the housings arranged on the base 1. In this condition, they are ready to be charged on the recharge station represented in FIG. 2.
- Connecting the power supplier to the grid and inserting it in the suitable connector of the base 1. The latter is automatically turned on. The recharging will start and only the state of the battery will be visible on the screen 4, reported in flashing manner;
- When the recharging is terminated, after about 4/5 hours, the symbol on the screen 4 of the battery will no longer flash;
- It is now possible to load the selected program from the base 1, so as to be able to use it in order to initiate a treatment.

The antennas 3 can work synchronously, loading the same program on both, or they can work separately, executing two distinct programs. In addition, the following textile components can be connected to the accessory 2, depending on the treatment that must be carried out:
- a total-body mat of about 50x100 cm;
- a face mask;
- a cervical band;
- a lumbar band;
- a knee/elbow/shoulder band;
- a glove for hand and wrist therapies;
- a vest;
- a magnetic mat 1 with dimensions of approximately 80x190 cm.

With respect to the technologies that are on the market, the dimensions of the apparatus are reduced and this allows an easy transportability. The physical characteristics of the product, in fact, are reported in Table 2.

**Table 2. Physical characteristics.**

| Dimensions | Range |
|---|---|
| Height | 22÷26 cm |
| Width | 26÷33 cm |
| Depth | 16÷20 cm |
| Weight | 3÷6kg |

With reference to FIG. 2, the wireless recharge station, also known as induction recharge, is a practice, wireless mode for recharging the electronic devices. The connection to a wireless battery charger is quicker and easier than that with cables. It is necessary to position the device depicted in FIG. 1 on the suitable space 6 which carries out the role of battery charger and the recharging will start instantly, without the need for external cables. On the wireless station, an acoustic signal is also present that is manifested when the device is inserted in the suitable space 6 and the battery charge level is shown on the LED 7, so as to signal the start and end of the recharging. The acoustic signal is newly repeated when the device is removed from the space 6 and the LED 7 is colored gray, signaling that there are no devices connected. In addition, the wireless battery charger protects the device from possible damage caused by repeated connections and disconnections of the recharging cables. In addition, the wireless station is certainly stronger than any cable, which is often ruined over time due to numerous wire signs. Finally, it is clear that modifications, additions or variations that are obvious for a man skilled in the art can be made to the invention described up to now, without departing from the protective scope that is provided by the enclosed claims.

## Claims

1. Magnetic field medical device constituted by intelligent antennas, **characterized in that** it employs a double therapy simultaneous with variable multifrequency and bipolar magnetic pulses in the field of magnetotherapy, said machinery being provided with:
- 100-240 Vac variable power supply;
- oscillation frequency 6-100 Hz (low frequency) 100-5000 Hz (high frequency);
- maximum power up to 200 Gauss per applicator;
said device comprising at least:
- a grid-powered base (1) with keyboard made of polycarbonate, function keys and backlit screen (4);
- a pair of antennas (3) power supplied with rechargeable internal battery;
- a plurality of textile accessories, adapted to be reversibly connected to said medical device and to adhere to a particular area of the body of a patient in order to treat it with the therapy provided by said medical device;
- a connection accessory (2) adapted to be reversibly connected with at least a textile accessory of said plurality of textile accessories provided as equipment;
- a lithium battery up to 3.7 V 900 mAh, duration of autonomy up to at least 90 minutes and in continuous supply;
- at least a pair of connection cables with said textile accessories (respectively 1.5 and 2.5 m).

2. Magnetic field medical device constituted by intelligent antennas, according to the preceding claim 1, **characterized in that** it is a wireless apparatus with communication range between base and local devices equal to at least 10 meters, in a normal living/housing condition.

3. Magnetic field medical device constituted by intelligent antennas, according to any one of the preceding claims, **characterized in that** it has dimensions equal to about 26÷33 cm of width, 22÷26 cm of height, 16÷20 cm of depth and weight comprised between 3÷6kg.

4. Magnetic field medical device constituted by intelligent antennas, according to any one of the preceding claims, **characterized in that** it comprises at its interior, beyond a rechargeable battery, also a wireless recharge station of variable dimensions comprised between 58÷64 cm of diameter and rather small weight comprised between 2÷4 kg; said recharge station allows the device to be recharged when it is not used, in only 4-5 hours.

5. Magnetic field medical device constituted by intelligent antennas, according to any one of the preceding claims, **characterized in that** it comprises at least: a USB port adapted to receive software updates, at least 100 stored protocols, at least 200 storable in the internal memory and at least 200 in a suitable Smart card.

6. Magnetic field medical device constituted by intelligent antennas, according to any one of the preceding claims, **characterized in that** it comprises alarms and safety equipment, reported hereinbelow:
- an acoustic signal for activating the device for each second of treatment, possibly deactivatable;
- an acoustic signal for therapy stoppage;
- calendar and clock;
- language change with at least 6 available languages;
- a timer to be set at the start of each therapy in order to define the duration, expressed in minutes;
- warnings and alarms relative to the state of the device, comprising at least: warning relative to the temperature of the environment in which the device is preserved when outside pre-established standards, optimal temperature during operation comprised between +15°C and +35°C and humidity between 45% and 75%, atmospheric pressure between 860 hPa and 1060 hPa, temperature of preservation and transportation at ambient temperature comprised between +5°C and +50°C; warning light in the case of activated device; alarm by means of a sensor which detects the presence of a heat source in proximity to the device, such as solar rays or heaters, and consequent alarm for instantaneous activity stoppage due to overheating danger;
- interlocking;
- emergency key for stopping therapy;
- an antifreeze sensor in order to prevent the temperature from falling below 5°C;
- function for controlling the emitted power;
- self-diagnosis.

7. Magnetic field medical device constituted by intelligent antennas, according to any one of the preceding claims, **characterized in that** it comprises an application for smartphones and tablets which connects the professional with an international network of doctor colleagues, presently active on the iOS and Android operating systems.

8. Magnetic field medical device constituted by intelligent antennas, according to any one of the preceding claims, **characterized in that** said plurality of textile accessories comprises at least:
- a total-body mat, about 50x100 cm;
- a face mask;
- a cervical band;
- a lumbar band;
- a knee/elbow/shoulder band;
- a glove for hand and wrist therapies;
- a vest;
- a magnetic mat with dimensions of approximately 80x190 cm.
